# EUROPEAN PATENT APPLICATION

(11) **EP 4 193 848 A1**
(43) Date of publication of application: **14.06.2023**
(21) Application number: 22212987.6
(22) Date of filing: 13.12.2022
(51) Int. Cl.: A23L 33/105, A61K 36/31, A61P 15/10

(54) **COMPOSITION FOR USE IN THE TREATMENT OF ERECTILE DYSFUNCTION**

(30) Priority: 13.12.2021 IT 202100031175
(71) Applicant: Anvest Group S.r.l., 20152 Milano (IT)
(72) Inventor: ALFIERI, Sandro, 84083 Castel San Giorgio (IT); ALFIERI, Dario, 84086 Roccapiemonte (IT); ALFIERI, Roberto, 84086 Roccapiemonte (IT)
(74) Representative: Cattaneo, Elisabetta

(57) **Abstract**

The invention concerns a composition comprising a rocket extract, a carnitine extract, citrulline, a Tribulus Terrestris extract and a Turnera Damiana extract in the form of a medicament or dietary supplement useful in the treatment of erectile dysfunction.

## Description

### FIELD OF THE INVENTION

The present invention refers to a composition comprising extracts of natural ingredients for the treatment of erectile dysfunction.

### BACKGROUND ART

The mechanism of erection is related to the vascularization of the penis and is regulated by the flow of blood in the cavernous bodies (the dilatable cylinders that are within the penis). In flaccid conditions, blood is supplied to the penis through the arteries and outflows, in equal measure, through the veins. In erection this equilibrium situation is altered by an increase in blood supply to the penis and a simultaneous reduction in venous outflow. It is a kind of electro-hydraulic mechanism: following an erogenous stimulation (visual, tactile, or even imaginary) some brain areas are triggered, which through nerve fibres of the spinal cord stimulate the vascular structures of the penis. The result is a vasodilation with hyperflow of blood to the penis and consequent enlargement and state of rigidity, which is maintained until the moment of ejaculation. The spongy tissue of the penis contains smooth muscle, fibrous tissue, veins and arteries. The urethra runs along the lower part of the cavernous bodies and within the spongy body. The impulses started from the brain or from the local nerves cause the muscles of the two cavernous bodies, and the spongy body, to relax, causing the penis to expand and allowing the tunica albuginea to trap blood in the underlying cavernous bodies so as to support the erection. When the muscles of the penis contract to block the inflow of blood and open the channels for the outflow, it returns to the flaccid state. In physiological conditions, one of the main positive mediators of the erection phenomenon is constituted by Nitric Oxide (NO) which is released by the nerve terminals and then spread in the smooth muscle of the cavernous bodies, where it activates the enzyme guanylate cyclase, which synthesizes cGMP, an intracellular mediator that allows the relaxation of the smooth muscle of the cavernous bodies, leading to the appearance of penile tumescence and erection. The enzyme phosphodiesterase-5 (PDE-5), hydrolyzes cGMP, disrupting the activating signal. The action mechanism of PDE-5 inhibitors is characterized by the inhibition of the enzyme PDE-5 expressed mostly at the level of the cavernous bodies, preserving cGMP degradation, able to maintain the myorelaxation of the cavernous bodies and therefore to preserve and prolong erection.

The normal erectile function is the result of a coordinated interaction between vascular, endocrine, neurological, psychological and genitourinary elements. Five physiological phases of sexual function can be recognised.
- The first phase, that of sexual desire (libido), involves the influence of psychological and endocrine factors (androgens).
- The second phase is the one that then results in erection, a neurovascular event that is achieved through the interaction between the sympathetic and parasympathetic innervation at the level of the penis, the smooth and striated musculature of the cavernous bodies and of the pelvic floor and that entails a substantial arterial blood flow. The erection develops and is maintained thanks to the arterial inflow and thanks to the venoconstriction that prevents the outflow thereof. The neurotransmitters that come into play in the mechanism of the erection are NO (nitric oxide vasoactive polypeptide), acetylcholine and prostaglandins.
- The third phase is ejaculation, controlled by the parasympathetic nervous system and consists of the emission of seminal fluid.
- The fourth phase is that of orgasm, a cortical phenomenon in which the rhythmic contraction of the bulbocavernous and ischiocavernous muscles determines the feeling of pleasure.
- The fifth phase, that of detumescence, is the result of the constriction of the trabecular smooth muscle that allows the venous blood to drain with consequent return to the state of flaccidity.

Erectile Dysfunction (ED) means the inability to obtain and/or maintain the erection in order to allow a satisfactory sexual intercourse. It affects on average 12.8% of males aged > 18 years. The prevalence increases with advancing age up to almost 50% in subjects aged >70 years. It is estimated that in Italy more than 3,000,000 men suffer from erectile dysfunction. Unfortunately, the issue is adequately addressed only by a very small part of the men concerned at all ages often out of fear or shame. The simultaneous presence of these factors greatly amplifies the risk (up to 12 times). Erectile dysfunction is sometimes called impotence and can also be used to describe other problems that interfere with sexual intercourse and reproduction, such as lack of sexual desire, ejaculation problems, and orgasm. Sporadic and occasional episodes of erectile dysfunction happen to most men and this is to be considered as normal. The issue evolves towards pathological when this condition is prolonged over time and makes having a normal sexual intercourse difficult up to the point of the impossibility of achieving orgasm.

For years the psychogenic cause has appeared to be the most common cause of erectile dysfunction. The mechanisms by which the psychological factors can promote the onset of an erectile dysfunction are not yet fully known. Hyperactivity of the sympathetic nervous system, which occurs, for example, under stressful conditions, certainly plays a key role. A particular condition is represented by the so-called "performance anxiety", which has an inhibiting effect on the erection and is frequent among young people at their first sexual experience, when confronted with a new partner or after a first failure in sexual intercourse occurred. The endocrine diseases most closely related to the erectile dysfunction onset are hypogonadism and hyperprolactinemia. In hypogonadism there is a reduction in testosterone levels, which is accompanied by a decrease in sexual desire and a reduction in erectile activity. In men with hyperprolactinemia, on the other hand, there is an increase in prolactin, which leads to an erectile deficit associated with reduced libido and infertility. Among the most major causes for erectile dysfunction there are certainly those vascular (endothelial dysfunction), both arterial and venous in nature. In the first case the erectile deficit is caused by a reduced blood supply to the penis, while the venous pathologies prevent the blood from remaining trapped within the cavernous tissue of the penis. Atherosclerosis is certainly the most common cause of erectile dysfunction of arterial origin; other causes of vascular damage that should be reminded are surgery and radiotherapy of the pelvis. At the systemic level, cardiovascular diseases represent one of the main predisposing conditions for erectile dysfunction. Atherosclerosis certainly represents the most common cause for erectile dysfunction of arterial origin; other causes of vascular damage are due to surgery of the urogenital area (radical prostatectomy) and pelvic radiotherapy. At a systemic level, cardiovascular diseases are one of the main predisposing conditions for erectile dysfunction. In a subject with a cardiovascular disease of any kind the risk of developing an erectile deficit compared to a healthy male is more than doubled. High blood pressure, an extremely common condition in the general population, causes erectile dysfunction in about 20% of hypertensive men. Myocardial infarction results in an erectile deficit in more than 45% of patients and a higher frequency of erectile dysfunction is found in subjects with stroke or other cerebrovascular accidents (about 85%).

The role of nitrogen monoxide (NO, also called nitric oxide) in the tumescence of the penis is known. Nitric oxide, first discovered as a vasodilator produced by the endothelium lining blood vessels, can be produced by neurons, macrophages and other cells to perform different functions in the body. In the vascular system, NO released from the endothelium by the enzyme endothelial nitric oxide synthase (eNOS) induces vasodilation and increases blood flow. In the penile circulation, this function of the endothelium is supplemented by the release of NO from the nitrergic nerve endings within the penis by the neuronal enzyme (nNOS) in the nitrergic nerves. These enzymes metabolize L-arginine into NO and L-citrulline. Both isoforms are present in the penis, nNOS in the nerve terminals of the autonomic nerves of the penis (pelvic plexus, cavernous nerves and dorsal nerves) and eNOS in the endothelium of the cisterns and of the cavernous arteries. The tumescence of the penis is obtained by a collaboration of these two NOS signalling pathways, in response to sexual stimulation, which induces the activation of the nitrergic nerves, which release NO to induce vasodilation and myorelaxation. As a result the blood flow of the penis increases, creating a traction force on the endothelium that induces vasodilation, following the phosphorylation and activation of eNOS, which releases endothelial NO, amplifying and supporting the increase in penile blood flow. Any alteration in one of these NO signalling pathways can attenuate and reduce the penile blood flow, causing erectile dysfunction. Traumatic or metabolic lesions, for example in diabetes mellitus, reduce nNOS and/or eNOS activation, causing erectile dysfunction. Other mechanisms, which can negatively affect the maintenance of adequate NO levels in the penile tissues, are linked to the oxidative damage caused by excess free radicals or smoking. These conditions in fact lead to the production of ROS (reactive oxygen species) which represent the antithesis of the NOS (reactive nitrogen species) to which NO is part. It follows that high levels of ROS, induce low levels of NO with consequent increased risk of erectile dysfunction. There are numerous effective drugs to treat erectile dysfunction. Subjects with low testosterone levels benefit from testosterone replacement therapy. Other treatments include self-injection of vasodilators, such as alprostadil. Injecting this prostanoid vasodilator into the base or the shaft of the penis increases the inflow and the retention of blood of the penis to induce tumescence. Other vasodilators for injection include papaverine and phentolamine. However, it should be considered that this type of intervention must be performed shortly before intercourse and is decidedly invasive. These approaches have been overcome by the inhibition of the enzyme phosphodiesterase-5 (PDE-5 i), which increase penile blood flow by inhibiting the degradation of cyclic guanosine monophosphate (cGMP) by PDE-5, increasing and prolonging the relaxing effect on the vascular and trabecular smooth muscle of the cavernous bodies to favour blood inflow and subsequent erection.

The object of the present invention is therefore to provide a treatment for erectile dysfunction which is effective and which has good compliance.

### SUMMARY OF THE INVENTION

The inventors have found a combination of active substances that was able to effectively treat erectile dysfunction. In particular, the inventors have identified a combination of substances, some already known for the treatment of erectile dysfunction, but which combined produce a greater synergistic effect than the additive combination of known substances as will become evident from the experimental part.

The invention therefore concerns composition comprising a rocket, carnitine, citrulline extract, a Tribulus Terrestris extract and a Turnera Damiana extract. All active substances are in the form of extracts as will be detailed below.

According to the invention said substances can also be provided in combination with an extract of ginseng, taurine, zinc and vitamin E.

The invention also concerns the composition comprising a rocket, carnitine, citrulline extract, a Tribulus Terrestris extract and a Turnera Damiana extract for use as a medicament.

According to a further aspect the invention concerns the composition comprising a rocket, carnitine, citrulline extract, a Tribulus Terrestris extract and a Turnera Damiana extract for use in the treatment of erectile dysfunction.

The invention also concerns a supplement comprising the composition of the invention and excipients, preferably for use in the treatment of erectile dysfunction. The supplement of the invention is therefore employable in the treatment of erectile dysfunction, specifically as improvement of erectile dysfunction, wherein said improvement is selected from the group consisting of improvement of testicular functions, improvement of vaso-relaxing properties on the muscular system, antioxidant activity and normal reproduction and maintenance of normal testosterone levels.

Advantageous aspects of the composition and of the supplement of the invention, such as amount, posology and dosages will be indicated in detail in the detailed description and the surprising synergistic effects deriving therefrom will become evident from the next experimental part.

In an advantageous aspect of the invention the composition of the invention may be combined with at least one phosphodiesterase 5 inhibitor such as Sildenafil, Tadalafil, Vardenafil in the treatment of erectile dysfunction.

The composition of the invention may in fact be administered alone or in combination, i.e. as a co-therapeutic agent in a fixed-dose combination or in a combined therapy of separately formulated active ingredients, with at least one phosphodiesterase 5 inhibitor, such as for example Sildenafil, Tadalafil, Vardenafil for the treatment of erectile dysfunction.

In another aspect the invention concerns the use of the composition of the invention or of the supplement in a fixed-dose combination or in a combined therapy of separately formulated active ingredients, with at least one phosphodiesterase 5 inhibitor.

The composition, i.e. the supplement comprising the same, of the invention alone or advantageously in combination with at least one phosphodiesterase 5 inhibitor, such as for example Sildenafil, Tadalafil, Vardenafil turned out to be effective in the treatment of erectile dysfunction, in particular with respect to the symptoms associated with it.

In fact, the composition of the invention, i.e. the medicament or the supplement comprising the same, shows an excellent activity of inhibiting the enzyme phosphodiesterase-5 (PDE-5) also improving testicular functions, as well as altering DNA with excellent antioxidant abilities, in addition to preserving nitric oxide in penile tissues and maintaining normal testosterone levels.

### DESCRIPTION OF THE DRAWINGS

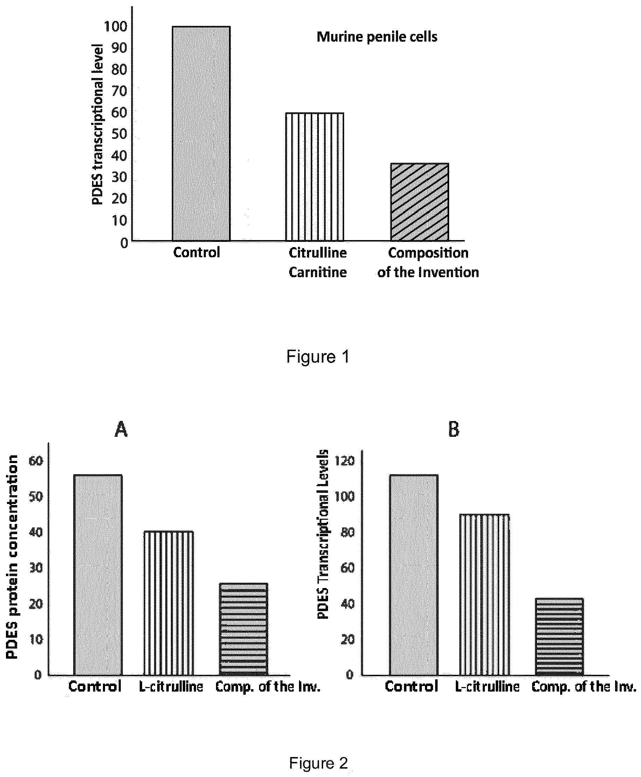

Figure 1 shows the effect of the treatment of the composition on type 5 phosphodiesterase activity on murine penile muscle cells compared to the action of different mixtures of the components. The vehicle alone was used as a further control. The data represent the mean ± SD with significance p<0.05, p<0.01.
Figure 2 shows the effect of the treatment of the composition of the invention on the activity of human type 5 phosphodiesterase on human muscle epithelium cells compared to the action of L-Citrulline alone (equivalent amount present in the composition). The vehicle alone was used as a further control. The data represent the mean ± SD with significance p < 0.05, p < 0.01.

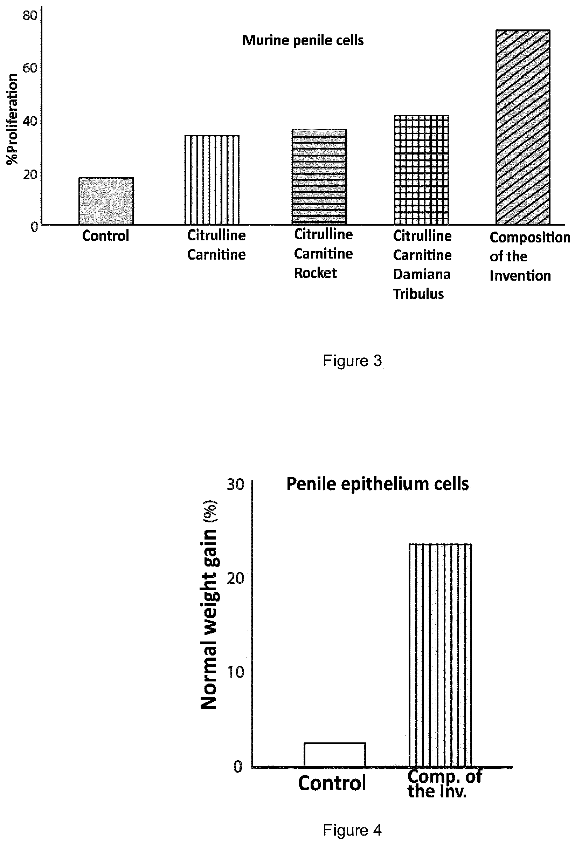

Figure 3 shows the effect on the proliferative activity of the murine penile muscle cells of the composition and of different mixtures of components, dosed in amounts equivalent to those present in the formula compared to the action of the medium alone of the culture medium alone. The data represent the mean ± SD with significance p < 0.05, p < 0.01.
Figure 4 shows the dose-dependent effect of the addition of different amounts of a solution consisting of an aqueous solution of the complete mixture and of the culture medium (1:1) to the weight increase of the muscle epithelial cells compared to the action of additions of only the culture medium used as a control. The data represent the mean ± SD with significance p < 0.05, p < 0.01.

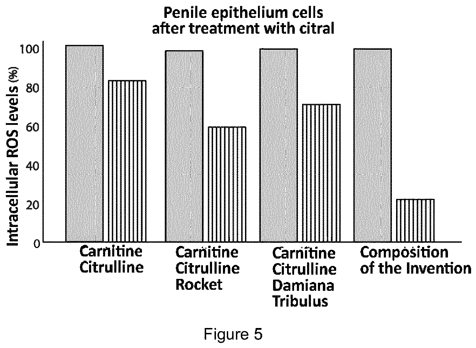

Figure 5 shows the flow cytometric analysis of intracellular ROS after the treatment with citral (100 µM) of the murine penile muscle cells after treatment with the composition and of different mixtures of components, dosed in amounts equivalent to those present in the composition compared to the action of the medium alone. The data represent the mean ± SD with significance ** p < 0.05, * ** p < 0.01.

### DETAILED DESCRIPTION OF THE INVENTION

The invention concerns composition comprising a rocket, a carnitine extract, citrulline, a Tribulus Terrestris extract and a Turnera Damiana extract.

The composition of the invention comprises a rocket extract, also known as Vasican eruca.

The composition of the invention thanks to the rocket extract contributes to the resolution of erectile dysfunction. Among the active substances of rocket, the extract of the invention comprising erucine is able to slowly release H2S (hydrogen sulfide) endogenous transmitter in gaseous form causing vasodilation and myorelaxation in tissues. The composition of the invention comprising Eruca vasicaria extract contributes to the improvement of testicular functions and spermatogenesis. It also induces an increase in testosterone levels and improves sperm activity. Icarin contained in rocket is a flavonoid that boasts multiple mechanisms of action including attenuation of DNA damage, correction of endothelial dysfunction, inhibition of proliferation and migration of smooth muscle cells. The main action of icarin lies in the inhibition of PDE-5, the mechanism underlying the treatment of erectile dysfunction.

The composition of the invention comprises the rocket extract, preferably in the form of rocket leaves extracted dry from 4 to 7%, more preferably to 5% of icarin in HPLC.

The composition of the invention preferably comprises the rocket extract in an amount from about 3.5% to about 12% by weight on the total weight of the composition, more preferably of about 7.5% by weight with respect to the total weight of the composition.

The composition of the invention preferably comprises the rocket extract as unitary dose per administration unit from about 100 g to about 300 mg, more preferably about 200 mg.

The composition of the invention comprises carnitine. The efficacy of the composition of the invention comprising carnitine in the treatment of erectile dysfunction has also been shown in its vascular and neuro-protective function. In particular, the action of carnitine in the composition of the invention on the muscle tissue allows to maintain blood in the penile area, slowing down the outflow in the second phase of the erection. Carnitine is also a muscle proenergetic, that is, it can provide the muscle tissue with the energy it needs by supplying energy from the beta-oxidation of lipids within the mitochondria. It, in fact, is responsible for transporting fatty acids within the mitochondria for their use for energy purposes. The composition of the invention comprises carnitine, preferably in the form of L-carnitine.

The composition of the invention preferably comprises carnitine in an amount from about 7% to about 25% by weight on the total weight of the composition, more preferably of about 18.7% by weight with respect to the total weight of the composition.

The composition of the invention preferably comprises carnitine as unitary dose per administration unit from about 200 g to about 600, more preferably about 500 mg.

The composition of the invention also comprises citrulline, preferably L-citrulline. The pharmacokinetic advantage of L-Citrulline is the absence of metabolization by the liver before entering the bloodstream, on the contrary, of L-Arginine subject to extensive hepatic and intestinal metabolism. The main organ responsible for converting L-citrulline is the kidney. Therefore, the release of L-Arginine is a propaedeutic factor of the blood L-Citrulline level.

The composition of the invention comprises citrulline, preferably in the form of L-citrulline. The composition of the invention comprising L-citrulline allows a synergism in the treatment of erectile dysfunction thanks to the metabolic conversion of L-citrulline into Arginine, involved in nitric oxide (NO) synthesis. At the penile level, through the stimulation of the enzyme guanylate cyclase, nitric oxide produces vasodilation, increasing the turgor of the cavernous bodies and contributing decisively to the erection of the penis. The biosynthesis of nitric oxide in endothelial cells and nerve terminals occurs mainly through the transformation of L-Arginine into L-Citrulline, operated by the enzyme NO-synthase (NOS). Newly formed Citrulline can in turn originate new Arginine restarting the cycle. Most L-Citrulline is converted into L-Arginine in the kidney and once in circulation, converted into L-Citrulline and nitric oxide, which in turn acts as a precursor to L-Arginine, repeating the cycle. However, the considerable presence of arginase at the intestine level, results in the conversion of a significant part of Arginine, administered orally, in Ornithine and Urea, inactivating the amino acid before absorption. Hepatic arginases also expose the absorbed portion of Arginine to a metabolization process that further reduces the systemically available portion for NO synthesis. Unlike arginine, L-Citrulline does not undergo systemic and presystemic metabolization, and its oral administration dose-dependently increases both Arginine and Citrulline plasma levels. At the same dosage and route of administration, Citrulline guarantees an increase in Arginine levels in the blood, almost double compared to those of a same dose of Arginine.

The composition of the invention preferably comprises citrulline, preferably L-citrulline, in an amount from about 18% to about 76% by weight on the total weight of the composition, more preferably of about 56.2% by weight with respect to the total weight of the composition.

The composition of the invention preferably comprises citrulline, preferably as L-citrulline, as unitary dose per administration unit from about 500 mg to about 2000 mg, more preferably about 1500 mg.

The composition of the invention also comprises the Tribulus terrestris extract.

The composition comprising Tribulus terrestris extract has properties capable of improving the detectable sexual behaviour with measurements of intracavernous pressure (ICP). The composition of the invention thanks to the presence of the tribulus represents a valid strategy for the treatment of erectile dysfunction since it allows:
- the direct conversion of the active substance present in Protodioscin PTN extract to dehydroepiandrosterone (DHEA) precursor of androgens and oestrogens.
- the increase in DHEA after administration of Tribulus extract, causes an antagonistic effect on gamma aminobutyric acid (GABA) thus facilitating sexual function;
- the regulation of the mechanism involving the NO / NO synthase pathway
The composition of the invention comprises the tribulus terrestris extract, preferably in the form of dry extract comprising 40% saponins.

The composition of the invention preferably comprises the tribulus terrestris extract, in an amount from about 1.8% to about 7.5% by weight on the total weight of the composition, more preferably of about 3.7% by weight with respect to the total weight of the composition.

The composition of the invention preferably comprises the tribulus terrestris extract, as unitary dose per administration unit from about 50 mg to about 200 mg, more preferably about 100 mg.

The composition of the invention also comprises Turnera damiana extract, preferably in the form of dry extract with ratio D:E = 4:1 (4 kg of plant in 1 kg of extract).

The composition of the invention thanks to the Turnera Damiana extract performs an aphrodisiac effect improving sexual behaviour in the men with erectile dysfunction. Its active substances, including arbutin, caffeine, and flavonoids, have a favourable activity for sexual function by reducing post-ejaculation latency time and increasing sexual desire. The action turned out to be aimed at the nerves of the pelvic-sacral parasympathetic. The composition of the invention thanks to the presence of the turnera damiana, which comprises visceral effector fibres that stimulate intestinal peristalsis of the descending, ileopelvic colon and rectum, causes the contraction of the detrusor muscle of the bladder and causes the release of the rectal and bladder sphincters; they also have vasodilatory action on the cavernous bodies of the penis and clitoris favouring erection.

The composition of the invention comprises Turnera Damiana extract, preferably in the form of dry extract with ratio D:E = 4:1 (4 kg of plant in 1 kg of extract). The composition of the invention preferably comprises Turnera Damiana extract in an amount from about 1.8% to about 7.5% by weight on the total weight of the composition, more preferably of about 3.7% by weight with respect to the total weight of the composition.

The composition of the invention preferably comprises Turnera Damiana extract, as unitary dose per administration unit from about 50 mg to about 200 mg, more preferably about 100 mg.

The composition of the invention may also comprise further ingredients which improve its activity.

The composition of the invention may also comprise an arginine extract.

The composition of the invention preferably comprises also the ginseng extract. Panax Ginseng has been fully included among the useful phytotherapeutic remedies for different pathologies, but little emphasis is given to its action for erectile dysfunction. The composition of the invention comprising ginseng has tonic-rejuvenating properties, also on sexual function. The intake of the composition thanks to the ginseng results in:
- An increase in the intracavernous pressure, fundamental for erection;
- A protection of the endothelium from the damaging action of free radicals, thus counteracting the potential deleterious effects of cigarette smoking and stress;
- An increase in intramuscular concentrations of nitric oxide;
- A slowing of histological and functional degeneration of blood vessels.

In the advantageous form of the composition of the invention comprising the ginseng extract it is preferably a dried extract of leaves comprising about 80% of ginsenosides measured with HPLC.

The ginseng extract is comprised in the composition in an amount from about 1.8% to about 7.5% by weight on the total weight of the composition, more preferably of about 5.6% by weight with respect to the total weight of the composition.

The composition of the invention preferably comprises Turnera Damiana extract, as unitary dose per administration unit from about 50 mg to about 200 mg, more preferably about 150 mg.

The composition of the invention may also comprise vitamin E.

Vitamin E, thanks to its antioxidant nature, reduces oxidative stress and is therefore able to preserve NO from oxidative inactivation. The composition of the invention thus improves vasodilation and blood inflow to the penile area and vascular reactivity. Oxidative stress appears to play a key role in the genesis of erectile dysfunction. External processes that generate oxidative stress such as smoking and unhealthy living can strongly increase the production of reactive oxygen species ROS that reduce endothelial NO concentration. There is therefore a significant association between the production of free radicals and the erectile dysfunction. Advantageously the composition of the invention comprises from 0.7% to 1.9% by weight on the total weight of the composition, more preferably about 1.8% on the total weight of the composition. The composition of the invention preferably comprises vitamin E, as unitary dose per administration unit from about 20 mg to about 50 mg, more preferably about 50 mg.

The composition may preferably also comprise zinc.

The composition thanks to the presence of zinc carries out antioxidant activity in the management of free radicals, but above all it induces an increase in testosterone in a dose-dependent way. Zinc supplementation leads to a significant increase in testosterone levels, increasing libido and sexual appetite in addition to increased sperm motility.

Advantageously the composition of the invention comprises zinc from 0.1% to 0.6% by weight on the total weight of the composition, more preferably about 0.5% on the total weight of the composition. The composition of the invention preferably comprises zinc, as unitary dose per administration unit from about 5 mg to about 15 mg, more preferably about 15 mg.

The composition of the invention also comprises taurine.

Advantageously the composition of the invention comprises taurine from 0.9% to 2.8% by weight on the total weight of the composition, more preferably about 1.8% on the total weight of the composition. The composition of the invention preferably comprises taurine, as unitary dose per administration unit from about 25 mg to about 75 mg, more preferably about 50 mg.

Taurine is an essential amino acid useful for increasing muscle performance. The composition of the invention comprising Taurine finds its application in the treatment of erectile dysfunction thanks also to its regulation of sperm motility and in the reduction of oxidative stress damage caused by the free radicals. In addition, the composition comprising taurine increases libido and stimulates blood microcirculation, maximizing erection and its duration.

The composition of the invention therefore turned out to be a selected and synergistic combination of active substances capable of treating erectile dysfunction.

According to a primary aspect of the invention the invention concerns the use of the composition comprising the composition of the invention for use as a medicament.

According to a further aspect the invention concerns the composition described further above for use in the treatment of erectile dysfunction.

The invention also concerns a supplement comprising the composition of the invention and excipients, preferably for use in the treatment of erectile dysfunction. The supplement of the invention is therefore employable in the treatment of the erectile dysfunction, specifically as improvement of the erectile dysfunction, wherein said improvement is selected from the group consisting of improvement of testicular functions, improvement of vaso-relaxing properties on the muscular system, antioxidant activity, preservation of nitric oxide in penile tissues and maintenance of normal testosterone levels.

The composition and the supplement for use in the treatment of the benign erectile dysfunction may preferably be administered by oral administration.

The compositions may then be in liquid, solid or semi-solid form and may be in the form of tablets, beads, drops, sticks, syrups, sachets.

The compositions of the invention are orally administrable in the form of tablets, capsules, granules, effervescent granules, syrups or the like.

They may also be dissolvable in water to form inhalable solutions.

The compositions in solid form may comprise one or more additives such as starches, sugars, cellulose, diluents, granulating agents, lubricants, binders, disintegrating agents, stabilizers, emulsifiers, texturizers, plasticizers, wetting agents, thickeners, coatings. All these excipients allow to obtain the supplement of the invention in the desired pharmaceutical formulation.

The capsules may be coated with a gastro-resistant film, or they may be soft gel capsules.

Flavouring agents, preservatives, dyes or similar agents may also be present.

The active substances contained in the compositions subject-matter of the present invention can be combined or mixed as active substances and/or excipients known according to the pharmaceutical and food or nutritional industry techniques and prepared by methods known in the pharmaceutical or food industry.

The compositions object of the invention are also used as adjuvants in the therapies for the treatment of erectile dysfunction.

In an advantageous aspect of the invention the composition of the invention may be combined with at least one inhibitor of the enzyme phosphodiesterase-5 (PDE-5).

The composition of the invention may in fact be administered alone or in combination, i.e. as a co-therapeutic agent in a fixed-dose combination or in a combined therapy of separately formulated active ingredients, with at least one inhibitor of the enzyme phosphodiesterase-5 (PDE-5) such as for example Sildenafil, Tadalafil, Vardenafil for the treatment of erectile dysfunction.

In another aspect the invention concerns the use of the composition of the invention or of the supplement as a co-therapeutic agent in a fixed-dose combination or in a combined therapy of separately formulated active ingredients, with at least one phosphodiesterase 5 inhibitor.

Said use in a fixed-dose combination or in a combined therapy of separately formulated active ingredients with at least one phosphodiesterase 5 inhibitor is preferably in the treatment and in the improvement of erectile dysfunction in a fixed-dose combination or in a combined therapy of separately formulated active ingredients, wherein said improvement is selected from the group consisting of improvement of testicular functions, improvement of vaso-relaxing properties on the muscular system, antioxidant activity, preservation of nitric oxide in penile tissues and maintenance of normal testosterone levels.

The composition, i.e. the medicament or the supplement comprising the same, of the invention alone or advantageously in combination with at least one inhibitor of the enzyme phosphodiesterase-5 (PDE-5) turned out to be effective in the treatment of erectile dysfunction.

In fact, the composition of the invention, i.e. the medicament or the supplement comprising the same, shows an excellent inhibitory activity against the enzyme phosphodiesterase-5 (PDE-5).

Specifically, the product of the invention exhibited excellent activities of improvement of testicular functions, improvement of vaso-relaxing properties on the muscular system, antioxidant activity, maintenance of normal testosterone levels and inhibition of degradation of nitric oxide in penile tissues.

In particular, the composition of the invention is therefore useful as a dietary and nutritional supplement, as a medicament, for the prevention and the treatment of erectile dysfunction, in particular in the improvement of the symptomatology of erectile dysfunction.

The compositions object of the invention are therefore pharmaceutical, nutraceutical preparations or food supplements that can be introduced into the dietary regimen of an individual suffering from erectile dysfunction or having one or more of the symptoms thereof.

The composition subject-matter of the present invention is therefore a valid therapeutic tool for the management of the symptoms of erectile dysfunction.

In this sense, the composition of the invention also has a preventive action, since the drug therapy with the PDE-5 inhibitors has the limit of having to be taken a few hours before the intercourse due to their average low half-life (about 3-4 hours). In addition, the PDE-5 inhibitors only perform this action, an aspect that can represent a limitation in patients with endothelial dysfunction or diabetes, who already from the beginning maintain low levels of NO in penile tissues. The composition of the invention represents an excellent therapeutic strategy to contribute to the increase of NO levels thanks to the presence of its precursors, in addition to the maintenance of NO levels by inhibiting PDE-5 and testosterone levels.

The advantageous and preferable effect occurs when the dose of the composition of the present invention with respect to the single administration unit respects the following characteristics:
i) L-Citrulline is present in a dose from 500 mg to 2000 mg, preferably 1500 mg.
ii) L-Carnitine is present in a dose from 200 to 600 mg, preferably at 500 mg.
iii) Rocket is present in a dose from 100 to 300 mg, preferably of 200 mg
iv) Ginseng is present in a dose from 50 to 200 mg, preferably at 150 mg.
v) Tribulus is present in a dose from 50 to 200 mg, preferably at 100 mg.
vi) Damiana is present in a dose from 50 to 200 mg, preferably at 100 mg.
vii) Zinc is present in a dose from 5 to 15 mg, preferably 15 mg
viii) Vitamin E is present in a dose from 20 to 50 mg, preferably 50 mg
ix) Taurine is present in a dose from 25 mg to about 75 mg, more preferably about 50 mg

The synergism generated by the combination further above has showed its efficacy in enhancing the effects of its components which resulted being greater than the sum of the known effects of the individual active substances.

### EXPERIMENTAL PART

### Example 1: preparation of the composition of the invention

For the preparation of the composition of the invention and therefore of the final product, such as for example a supplement, the following were employed:
The composition of the invention was prepared using the method of geometric dilutions so as to obtain a homogeneous mixing of the powders in mortar. The composition was prepared with the following ingredients in the indicated amounts:
- Rocket extract having a content of D.E. Leaves. 5% Icarin. It was present in an amount of 200 mg.
- Carnitine was present in an amount of 500 mg
- Citrulline was present in an amount of 1500 mg.
- The dried extract of tribulus fruits having a content of 40% by weight of saponins with respect to the weight of the extract. Tribulus extract was present in an amount of 100 mg.
- The dry extract of Turnera Damiana leaves with D/e ratio 4:1. Turnera Damiana extract was present in an amount of 100 mg.

The composition further above could also comprise:
- The dry extract of Ginseng, leaves, having a content of 80% by weight of HPLC ginsenosides. Ginseng extract was present in an amount of 150 mg.
- Vitamin E, 50mg
- Zinc, 15 mg
- Taurine, 50 mg

The compositions of the invention could be formed by combining the ingredients further above in the amounts indicated, or by the amounts falling within the ranges indicated further above.

### Example 2 Evaluation of the composition of the invention.

The research was aimed at demonstrating how the composition of the invention was effective in the treatment of erectile dysfunction with a synergistic effect among the components. In particular, the in vitro tests carried out in the laboratory analysed the mechanisms of action commonly involved in erection such as the reduction of nitric oxide (NO) degradation, the inhibition of the enzyme phosphodiesterase-5 (PDE5) and the ability of the tissue to retain solutes to increase turgidity.

### Phosphodiesterase 5 inhibition on murine model (PDE5)

The studies on the effects of the present composition on the inhibition of type 5 phosphodiesterase were carried out by means of an ELISA test, using a PDE5 ELISA kit (ThermoFisher Scientific).

Murine penile muscle epithelium cells (Murine penile cell lines SAP1), after their culture in DMEM, 10% and FBS, were subjected to treatment with different mixtures of the components of the formula, and with the entire composition of the invention. The PDE5 levels were quantified using Immunoenzyme Assay Kits (ELISA) on the supernatant. The supernatant obtained from the same amount of cells treated with the different mixtures of components, in an amount equal to that present in the composition, that obtained from the same amount of cells treated with the composition, and from the same amount of untreated cells, used as a control, were subjected to immunoenzyme analysis. The experiments were carried out sixfold for each single experimental point reported.

Figure 1 shows the variation in transcriptional levels of PDE5 after treatment of the murine penile muscle cells. The results have showed how an aqueous solution containing the entire composition is capable of inhibiting type 5 phosphodiesterase in a significantly superior manner to the vehicle used as a control, and to the mixture of L-Citrulline and L-Carnitine, dosed in a ratio equivalent to that present in the composition. Specifically, the composition of the invention have showed an efficacy in inhibiting PDE5 levels higher than 65% with respect to the control and 35% with respect to L-Citrulline + L-Carnitine.

### Inhibition of human phosphodiesterase 5 (PDE5)

The studies on the effects of the composition on the inhibition of human type 5 phosphodiesterase were carried out by means of an ELISA test, using a PDE5 ELISA kit (ThermoFisher Scientific). The human muscle epithelium cells subjected to the treatment with the composition of the invention were homogenized in a 0.01 M PBS solution (pH 7.4) and subsequently centrifuged (3000 rpm, 4°C, 20 min). The PDE5 levels were quantified using Immunoenzyme Assay Kits (ELISA) on the supernatant. The supernatant obtained from the same amount of cells treated with L-Citrulline, in an amount equal to that present in the formula, and from the same amount of untreated cells, used as a control, were subjected to immunoenzyme analysis and the data used as a comparison. The experiments were carried out sixfold for each single experimental point reported.

The efficacy in inhibiting the production and transcription of human type 5 phosphodiesterase of the composition, with respect to the same amount present in the L-Citrulline mixture, has been tested using human muscle epithelium cells. Figure 2 shows the variation of PDE5 (A) and its transcriptional levels (B) after treatment of the epithelial cell. The results have showed how an aqueous solution containing the composition of the invention (0.5 mg/mL) is capable of inhibiting human type 5 phosphodiesterase in a significantly superior manner to the vehicle used as a control, and to the L-Citrulline dosed in a ratio equivalent to that present in the composition of the invention. Specifically, the composition has showed efficacy in inhibiting PDE5 levels higher than L-Citrulline by 25% and 43% with respect to the control. This difference rises further when reference is made to the transcriptional levels of PDE5, where the composition of the invention formula is more effective than L-Citrulline alone by about 60%.

### Cell Proliferation Studies of the murine penile muscle epithelium

The murine penile muscle epithelium cells (SAP1) were isolated, stained using a 5 µM solution of carboxyfluorescein succinimidyl ester and incubated at room temperature for 10 minutes. The epithelial cells were then treated with a solution (0.5 mg/mL) of the composition under study, of different solutions of mixtures composed of the individual components of the composition and a solution of culture medium used as a control. The cells thus treated were incubated for 48 h, then harvested and the percentage of proliferating cells in each condition was determined by cytofluorimetry.

As can be noted from Figure 3, the composition is able to positively and significantly stimulate the proliferation of the murine penile muscle epithelium cells. Specifically, the tests carried out were able to increase cell proliferation by about 62% with respect to the control. It should also be specified that the synergistic action of the entire composition on cell proliferation does not represent the simple addition of the individual contributions of each combination, but a synergy is established that allows surprising results to be achieved. In fact, the actions on cell proliferation of the combinations Carnitine + Citrulline, Citrulline + Carnitine + Rocket and Citrulline + Carnitine + Damiana + Tribulus do not exceed 40% in terms of cell viability. Moreover, it is not a negligible fact that the entire composition represents for the cells a vehicle that increases their proliferation capacity since the concentration and the number of herbal extracts could have represented a limit to cell growth due to problems of diffusibility of nutrients, solubilization of powders or synergistic antagonisms.

### Determination of the Induced Cell Turgor of the Human Muscle Epithelium

The human muscle epithelial cells were isolated from the culture medium and resuspended in PBS at a density of 1x107 cells/mL. The epithelial cells were then treated with a 1:1 solution of the complete mixture and of the culture medium and an equal volume of the solution of only the culture medium used as a control. After 24 h the wells emptied from the medium were weighed and compared with their weight before treatment. The weight obtained was then normalised with respect to the number of cells present in the well. The normalised weight increases of the cells treated with the mixture and those untreated were used as an indicator of increased membrane permeability and cell turgor.

Figure 4 shows the normalised weight increase of human muscle epithelium cells treated with different dosages of an aqueous solution, in which the composition of the present invention was present. Specifically, Figure 4 shows how the addition of the composition is able to increase permeability and retention of salts/nutrients by the treated cells by approximately 24% compared to the control. The increase in the capacity of the cells to retain salts and nutrients is closely related to the resolution of erectile dysfunction. In fact, when the cells are able to increase their internal osmotic balance (concentration of intra-cellular solutes) they can host significantly greater volumes of intra-cellular fluids. This is a clear application to the ability of the penile cavernous tissue to be supplied with blood with consequent maintenance of the erection.

### Measurement of ROS levels in murine penile muscle epithelium cells

The modulation of the intracellular level of ROS by the citral in murine penile muscle epithelium cells (SAP1) was measured using the fluorescent probe 2',7'-dichlorfluorescein-diacetate (DCFH-DA). The cells were treated with citral to induce the formation of free radicals in the form of ROS (reactive oxygen species). 30 minutes before treatment with citral a group of cells were treated with different solutions of mixtures composed of the individual components, in amounts equivalent to those present in the composition of the invention in order to verify the effect of the composition on the formation of reactive species ROS.

Figure 5 shows intracellular levels of reactive oxygen species ROS generated by oxidative stress due to addition of a citral solution to the cells. As shown by the data reported in Figure 5, this induction is significantly reduced when before the treatment with citral the cells were treated with the composition subject-matter of the present invention or with several of its components. This effect was maximum in the case of the entire composition, able to counteract the formation of ROS by about 85% with respect to the control, where the individual mixtures were able to reduce ROS levels at the intracellular level by a maximum of 40%. It should be pointed out that this ability to inhibit the formation of ROS is closely related to the management of the erectile dysfunction. In fact, the presence of ROS negatively affects the formation and the maintenance of adequate levels of nitric oxide NO, essential for penile and erection functionality. It follows that low levels of ROS at the penile cell level are equivalent to high concentrations of NO.

## Claims

1. A composition comprising a rocket, carnitine, citrulline extract, a Tribulus Terrestris extract and a Turnera Damiana extract.

2. The composition according to claim 1, wherein the rocket extract comprises icarin and erucine, preferably in the form of rocket leaves extracted dry from 4 to 7%, more preferably 5% of icarin measured with HPLC.

3. The composition according to claim 1 or claim 2, wherein the composition comprises the rocket extract in an amount from about 3.5% to about 12% by weight on the total weight of the composition, preferably of about 7.5% by weight with respect to the total weight of the composition.

4. The composition according to claim 1 wherein the rocket extract as unitary dose per administration unit is in the range from about 100 g to about 300 mg, more preferably about 200 mg.

5. The composition according to any one of claims 1 to 4, wherein carnitine, preferably in the form of L-carnitine, is in an amount from about 7% to about 25% by weight on the total weight of the composition, preferably of about 18.7% by weight with respect to the total weight of the composition.

6. The composition according to any one of claims 1 to 4, wherein carnitine, preferably in the form of L-carnitine, is present as unitary dose per administration unit from about 200 g to about 600, preferably about 500 mg.

7. The composition according to any one of claims 1 to 6, wherein citrulline, preferably L-citrulline, is present in an amount from about 18% to about 76% by weight on the total weight of the composition, preferably of about 56.2% by weight with respect to the total weight of the composition.

8. The composition according to any one of claims 1 to 6, wherein citrulline, preferably L-citrulline, is present in an amount as unitary dose per administration unit from about 500 mg to about 2000 mg, preferably about 1500 mg.

9. The composition according to any one of claims 1 to 8, wherein the tribulus terrestris extract is in an amount from about 1.8% to about 7.5% by weight on the total weight of the composition, more preferably of about 3.7% by weight with respect to the total weight of the composition.

10. The composition according to any one of claims 1 to 8, wherein the tribulus terrestris extract is in an amount as unitary dose per administration unit from about 50 mg to about 200 mg, more preferably about 100 mg.

11. The composition according to any one of claims 1 to 10, wherein the Turnera Damiana extract, preferably in the form of dry extract with ratio D:E = 4:1 (4 kg of plant in 1 kg of extract), is in an amount from about 1.8% to about 7.5% by weight on the total weight of the composition, preferably of about 3.7% by weight with respect to the total weight of the composition.

12. A composition according to any one of claims 1 to 11 for use in the treatment of erectile dysfunction.

13. A supplement comprising the composition according to any one of claims 1 to 11 and excipients.

14. Use of the composition according to any one of claims 1 to 11 or of the supplement of claim 14 in the improvement of erectile dysfunction, wherein said improvement is selected from the group consisting of improvement of testicular functions, improvement of vaso-relaxing properties on the muscular system, antioxidant activity, preservation of nitric oxide in penile tissues and maintenance of normal testosterone levels.

15. Use of the composition according to any one of claims 1 to 11 or of the supplement according to claim 13 in the treatment and improvement of erectile dysfunction in a fixed-dose combination or in a combined therapy of separately formulated active ingredients, with at least one inhibitor of the enzyme phosphodiesterase-5 (PDE-5), wherein said improvement is selected from the group consisting of improvement of testicular functions, improvement of vaso-relaxing properties on the muscular system, antioxidant activity, preservation of nitric oxide in penile tissues and maintenance of normal testosterone levels.
